# EUROPEAN PATENT SPECIFICATION

(11) **EP 1 443 864 B1**
(45) Date of publication and mention of the grant of the patent: **03.10.2007**
(21) Application number: 02780523.3
(22) Date of filing: 24.10.2002
(51) Int. Cl.: A61B 17/70

(54) **ADJUSTABLE TANDEM CONNECTORS FOR CORRECTIVE DEVICES FOR THE SPINAL COLUMN AND OTHER BONES AND JOINTS**
EINSTELLBARE TANDEMVERBINDUNGSELEMENTE FÜR KORREKTURVORRICHTUNGEN FÜR DIE WIRBELSÄULE UND ANDERE KNOCHEN UND GELENKE
CONNECTEURS EN TANDEM REGLABLES DESTINES A DES DISPOSITIFS DE CORRECTION DE LA COLONNE VERTEBRALE ET D'AUTRES OS ET ARTICULATIONS

(30) Priority: 31.10.2001 US 334771 P
(43) Date of publication of application: 11.08.2004
(73) Proprietor: Zimmer Spine, Inc., Minneapolis, MN 55439-2029 (US)
(72) Inventor: ROYCHOWDHURY, Suranjan, Plymouth, MN 55441 (US); NGUYEN, Duy, Woodbury, MN 55125 (US); COTA, Jeffrey, St. Louis Park, MN 55426 (US)
(74) Representative: Humphreys, Ceris Anne
(86) International application number: PCT/US2002/034031
(87) International publication number: WO 2003/037200

(56) References cited:
- WO-A-00/59387
- US-A- 5 885 284

## Description

The present invention relates generally to devices for correcting the spinal column and other bones and joints, and more specifically to connectors for such devices.

### Background of the Invention

The bones and connective tissue of an adult human spinal column consist of an upper portion (the cervical, thoracic, and lumbar regions) having more than 20 discrete bones, and a lower portion which consists of the sacral bone and the coccygeal bodies. The bones of the upper portion are generally similar in shape, the size of the bones progressively varying from small to large downwardly along the spine.

The vertebrae are coupled to one another by a tri-joint complex consisting of an anterior disc and the two posterior facet joints, the anterior discs of adjacent bones being cushioned by cartilage spacers referred to as intervertebral discs. Referring now to **Figures 1, 2** and **3** (which are reproductions of Figures 1-3 of U.S. Patent No. 5,885,284, top, lateral, and posterior views, respectively, of typical vertebral bones of the spinal column are shown. The spinal cord is housed in the central canal **10,** protected from the posterior side by a shell of bone called the lamina **12.** The lamina **12** has three large protrusions. Two of these extend laterally from the side ends thereof and are referred to as the transverse processes **14.** The third extends back and down from the center of the lamina and is called the spinous process **16.** The lamina **12** defines an arched shape about the posterior of the spinal cord, the arched shape having lateral portions **13a, 13b** which are generally straight, and which meet beneath the spinous process **16** at a curved surface **15.**

The anterior portion of the spine comprises a set of generally cylindrically shaped bones which are stacked one on top of the other. These portions of the vertebrae are referred to as the vertebral bodies **20,** and are each separated from the other by the intervertebral discs **22.** Pedicles **24** are bone bridges which couple the anterior vertebral body **20** to the corresponding lamina **12** and transverse and spinous processes **14,16.**

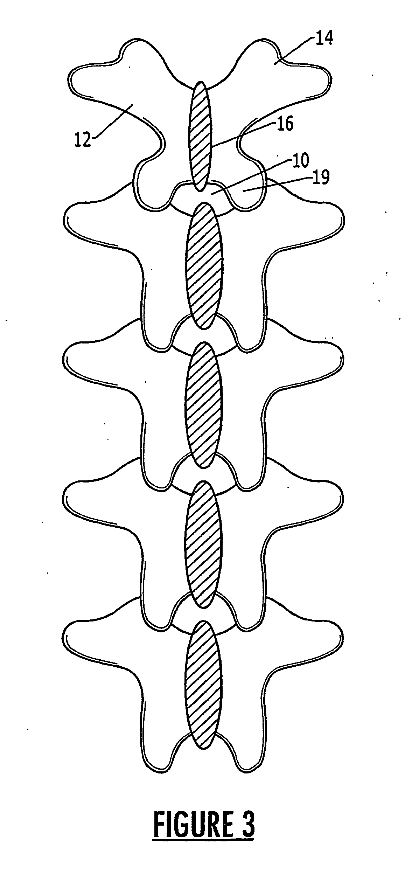

Referring specifically to **Figure 3,** the stacking of vertebrae is shown from the posterior. As can be seen in **Figure 3,** each vertebra is coupled to the one above and below via facet joints **19** on either side of an opening into the spinal canal **10.**

In its entirety, the spinal column is highly complex in that it houses and protects critical elements of the nervous system which have innumerable peripheral nerves and arterial and venous bodies in close proximity. In spite of these complexities, the spine is a highly flexible structure, capable of a high degree of curvature and rotation through a wide range of motion. Genetic or developmental irregularities, trauma, chronic stress, tumors, and disease, however, can result in spinal pathologies which either limit this range of motion or threaten the critical elements of the nervous system housed within the spinal column.

Such pathologies may be treated by a wide variety of therapeutic interventions, including immobilization of one or more vertebrae. A variety of systems have been proposed which achieve this immobilization by implanting artificial assemblies in, or on, the spinal column. These assemblies may be classified by their position relative to the spine as anterior, posterior, or lateral implants. Anterior and lateral assemblies generally comprise short structures which support only a few adjacent vertebral bodies. Conversely, posterior implants often comprise pairs of elongate vertically disposed rods for stabilizing both short and long segments of the spine. Typically, such posterior rods are coupled to the back of the spinal column via hooks which slip under the lamina, means for attachment to the transverse process, and/or by screws which are inserted through the pedicle (often termed "pedicle screws").

In some instances it may be desirable to provide enhanced torsional rigidity to the rods. In such instances, cross-linking devices or connectors which couple the rods together transverse to the axes of the rods are typically employed. Exemplary devices and connectors are illustrated in U.S. Patent Nos. 5,885,284 to Errico et al., 5,084,049 to Asher et al., 5,752,955 to Errico et al., 6,136,003 to Hoeck et al., 6,113,600 to Drummond et al., PCT Publication No. WO 00/59387, and 5,368,594 to Martin et al. In other instances, such as revision or extension procedures, it may be desirable to utilize a connector to connect new hardwared to the existing rods or plates. Exemplary devices and connectors commercially available from DePuy Acromed (e.g., Moss Miami axial connectors and Isola Aval rod connectors) and Sofamore Danek (e.g., CROSSLINK ® multi-span plates and offset plates, TSRH offset plates, and CD Horizon axial/domino connectors). In considering the design of a connector, issues include its size (because the connector is implanted in the body near the spine, it should occupy a relatively small volume in order that it be comfortable and non-intrusive for the patent) and its ease of implantation (which can encompass both the ease of attaching an individual rod to the connector and its orientation relative to the patient and the doctor during surgery). Because (a) there are a number of different surgical procedures in which these connectors are employed and (b) different surgeons have different preferences for connector configurations, it is desirable to provide new connector configurations that can meet individual surgeons' needs.

### Summary of the Invention

Connector embodiments of the present invention are configured to address different surgical needs and techniques for interconnecting multiple bone fixation devices (such as rods or plates between vertebrae). As a first aspect, the present invention is directed to a connector as defined in claim 9 that comprises: first and second mating members, each of the members including a body portion, a mating projection and a recess adapted to engage a respective one of at least two bone fixation rods; first and second retaining members; and a fastener. The body portions of the mating members include an aperture having a longitudinal axis that is generally perpendicular to longitudinal axes of the rods, and the mating projections of the first and second mating members include an aperture, the mating projection of the first mating member overlying the mating projection of the second mating member such that their respective apertures are generally axially aligned. The first and second retaining members are inserted into, respectively, the body portion apertures of the first and second mating members to engage a respective rod. The fastener is inserted through the mating projection apertures of the first and second mating members. When the fastener is in a tightened condition, the first and second mating members are prevented from relative rotation, and when the fastener is in a loosened condition, the first and second mating members are free to rotate about an axis of rotation that is generally parallel to the longitudinal axes of the body portion apertures of the first and second mating members. In this configuration, the connector can be manipulated in the coronal plane of the subject to interconnect non-parallel rods, and the rods can be secured and the connector tightened easily by a surgeon from above the spine. Each of the body portion recesses has a center, which centers are positioned between about 8,9mm (0.35 inches) and about 30,5mm (1.2inches) from each other.

There is further provided an apparatus for connecting vertebrae of a subject as defined in claim 1, comprising:
at least two rods, each of the rods defining a longitudinal axis; and
a connector that interconnects the rods, the connector comprising:
   first and second mating members, each of the members including a body portion, a mating projection and a recess adapted to engage a respective one of the rods, the body portion including an aperture having a longitudinal axis that is generally perpendicular to the longitudinal axes of the rods, each of the mating projections of the first and second mating members including an aperture, the mating projection of the first mating member overlying the mating projection of the second mating member such that their respective apertures are generally axially aligned;
   first and second retaining members inserted into, respectively, the body portion apertures of the first and second mating members to engage a respective rod; and
   a fastener inserted through the mating projection apertures of the first and second mating members;
wherein, when the fastener is in a tightened condition, the first and second mating members are prevented from relative rotation, and when the fastener is in a loosened condition, the first and second mating members are free to rotate about an axis of rotation that is generally parallel to the longitudinal axes of the body portion apertures of the first and second mating members. Each of the body portion recesses has a center, which centers are positioned between about 8,9 mm (0.35inches) and about 30,5mm (1.2 inches) from each other.

Contacting surfaces on the mating projections of the first and second mating members may include nesting topography that inhibits relative rotation of the first and second mating members when the fastener is in the tightened condition.

The mating projection apertures may be configured such that, when the fastener is in the loosened condition, the first and second mating members are free to translate relative to each other along a translation axis that is perpendicular to the axis of rotation and the longitudinal axes of the fingers of the first and second mating members.

The first and second retaining members may be threaded fasteners accessible from a first side of the connector, wherein the fastener is accessible from the first side of the connector.

Each of the first and second mating members may further comprise a finger, the fingers and the body portions of the first and second mating members together forming, respectively, the recesses of the first and second mating members.

Each of the body portions may have an upper surface, and the mating projections of the first and second mating members may be located at different first and second distances from their respective body portion upper surfaces.

Each of the body portion recesses may have a center, wherein the recess centers may be positioned at substantially the same distance from the body portion upper surfaces.

Each of the body portions of the first and second mating members may have a cutaway portion that is complimentary to and nested with an end of the mating projection of the other of the first and second mating members.

### Brief Description of the Figures

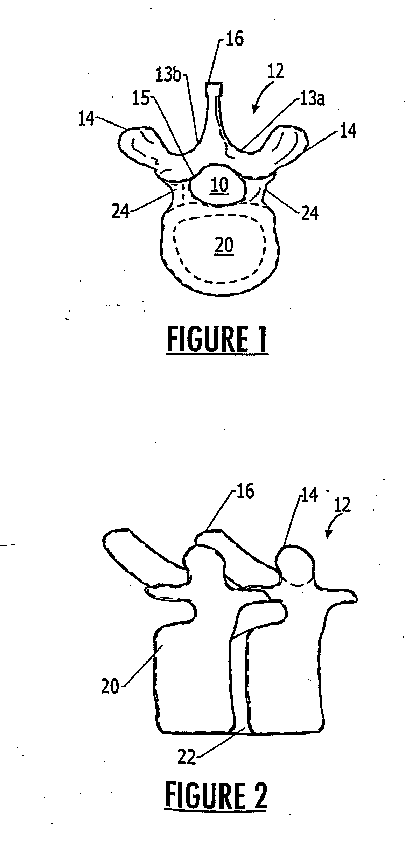

**Figure 1** is a top view of a vertebra of the human spine.
**Figure 2** is a side view of a series of vertebrae of the human spine.
**Figure 3** is a posterior view of a series of vertebrae of the human spine.
**Figure 4** is a perspective view of a connector embodiment of the present invention joining two bone fixation rods.
**Figure 5** is an exploded perspective view of the connector embodiment of **Figure 4.**
**Figure 6** is a perspective view of a connector which is not port of the present invention joining two bone fixation rods.
**Figure 7** is an exploded perspective view of the connector example of **Figure 6.**
**Figure 8** is a perspective view of an additional connector which is not port of the present invention joining two bone fixation rods.
**Figure 9** is an exploded perspective view of the connector example of **Figure 8.**
**Figure 10** is a perspective view of a further connector embodiment of the present invention joining two bone fixation rods.
**Figure 11** is an exploded perspective view of the connector embodiment of **Figure 10.**
**Figure 12** is a perspective view of still another connector which is not port of the present invention joining two bone fixation rods.
**Figure 13** is an exploded perspective view of the connector example of **Figure 12.**
**Figure 14** is a perspective view of yet another connector which is not port of the present invention joining two bone fixation rods.
**Figure 15** is an exploded perspective view of the connector example of **Figure 14.**
**Figure 16** is a perspective view of another connector which is not port of the present invention joining two bone fixation rods.
**Figure 17** is an exploded perspective view of the connector example of **Figure 16.**

### Detailed Description of the Invention

The present invention will now be described more fully hereinafter, in which preferred embodiments of the invention are shown. This invention may, however, be embodied in different forms and should not be construed as limited to the embodiments set forth herein. Rather, these embodiments are provided so that this disclosure will be thorough and complete, and will fully convey the scope of the invention to those skilled in the art. In the drawings, like numbers refer to like elements throughout. Thicknesses and dimensions of some components may be exaggerated for clarity.

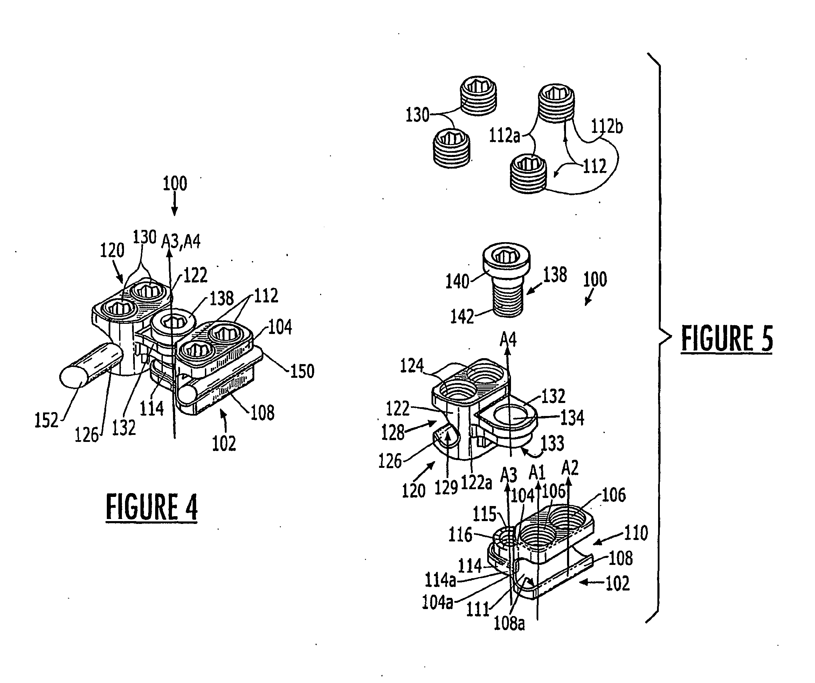

Turning now to the figures, a connector, designated broadly at **100,** is illustrated in **Figures 4** and **5.** The connector **100** includes a first mating member **102,** a second mating member **120,** and a bolt **138.** These components are described in detail below.

The first mating member **102** includes a body portion **104,** a finger **108,** and a mating projection **114.** The body portion **104** is generally wedge-shaped and includes a pair of apertures **106,** each of which has a respective longitudinal axis **A1, A2.** The finger **108** extends from one end of the body portion **104.** The free end of the finger **108** and the lower edge of the body portion **104** form an opening **110** that leads to a recess **111** defined by an arcuate upper surface **108a** of the finger **108** and a substantially flat lower surface **104a** of the body portion **104.** Two set screws **112** extend through the apertures **106;** the heads **112a** of the set screws **112** extend above the body portion **104** when in a loosened condition, and the shanks **112b** of the set screws **112** extend into the recess **111.** The mating projection **114** extends away from a lower region **114a** of the body portion **104** in a direction generally opposite that of the finger **108.** The mating projection **114** has serrations **115** on its upper surface. A threaded aperture **116** extends through the mating projection **114;** the aperture **116** includes a longitudinal axis **A3** that is generally parallel with the axes **A1, A2.** The mating projection **114** includes a cutaway portion **118.**

The second mating member **120** is the mirror image of the first mating member **102** with the exception of the location and configuration of its mating projection. More specifically, the second mating member **120** includes a body portion **122** with threaded apertures **124,** a finger **126** that, with the body portion **122,** forms an opening **128** into a recess **129,** and set screws **130** that extend through the apertures **124.** The mating projection **132** of the second mating member **120** extends from an upper region **122a** of the body portion **122** in a direction generally opposite that of the finger **126.** The mating projection **132** includes serrations **133** on its lower surface that are configured to mate with the serrations **115** of the mating projection **114** of the first mating member **102.** A non-threaded aperture **134** extends through the mating projection **132** and has a longitudinal axis **A4** that is substantially coincident with the axis **A3.** When the second mating member **120** is attached to the first mating member **102,** the mating projection **132** overlies the mating projection **114;** also, the mating projection **132** is complimentary to and nests within the recess **109** of the first mating member **102,** and the mating projection **114** is complimentary to and nests within the recess **135** of the second mating member **120.**

The bolt **138** has a head **140** and a threaded shank **142.** The shank **142** extends through the aperture **134** and is threadedly received in the aperture **116,** such that the head **140** resides above the mating projection **132.**

As can be seen in **Figure 4,** the connector **100** can be attached to two rods **150,152** that have been mounted to vertebrae of a subject. In most instances, the connector **100** will be oriented such that the head **140** of the bolt **138** faces away from the spine. After the attachment of the rods **150, 152** to respective vertebrae, the connector **100** is positioned so that one of the rods **150** is engaged within the recess **111,** and the other rod **152** is engaged within the recess **129.** In each instance, the rods **150, 152** can be inserted into their respective recesses **111, 129** through the openings **110, 128** located on the sides of the first and second mating members **102, 120.** The bolt **138** should be in a loosened condition, thereby enabling the first and second mating members **102, 120** to rotate relative to each other about the axes **A3, A4** (i.e., the first and second mating members **102, 120** are free to rotate relative to each other within the coronal plane of the subject) to accommodate non-parallel rods **150,152.**

After the rods **150, 152** are positioned within their respective recesses **111, 129,** they can be secured therein through the tightening of the set screws **112, 130**. Notably, the set screws **112, 130** are oriented so that the tightening heads thereof face the same direction (i.e., away from the spine) as the head **140** of the bolt **138.** This orientation typically will cause the heads of the set screws **112, 130** to face the surgeon, thereby facilitating tightening of the set screws **112, 130.**

After the set screws **112, 130** are tightened, the bolt **138** can then be tightened into the aperture **116** to force the mating projections **114, 132** together. The compression of the mating projections **112, 130** causes the serrations **115, 133** to nest and mate, thereby preventing relative rotation of the first and second members **102, 120.** Like that of the set screws **112, 130,** the head **140** of the bolt **138** faces the surgeon to facilitate tightening.

Of course, the connector **100** can be attached to the rods **150, 152** in a different sequence of steps than that described above. For example, one or both of the rods can be attached to the connector **100** prior to attachment of the rod to a vertebra of the subject. Also, the bolt **138** may be tightened prior to the tightening of the set screws **112, 130,** or the bolt **138** and/or the set screws **112, 130** may be tightened to less than full torque during insertion, then tightened to a higher torque magnitude after all components have been inserted. The skilled artisan will understand that other sequences of steps for insertion may also be suitable.

Notably, the connector **100** is configured such that the centers of the recesses (i.e., the locations where the centers of the rods **150, 152** reside within the recesses **111, 129)** are located at substantially the same depth relative to the top surfaces of the body portions **104, 122.** This results from the offset relationship of the mating projections **114, 130,** in which the mating projection **114** extends from the lower region of the body portion **104,** while the mating projection **132** extends from the upper region of the body portion **122.** Because the centers of the rods **150, 152** are located at approximately the same depth in the subject, the connector **100** can be recessed farther from the subject's dorsal skin surface than some prior connectors that lack this offset design.

Each of the mating members **102, 120** is typically formed as a unitary component, preferably of titanium, titanium alloys (like Ti-6Al-7Nb), nickel titanium alloys, cobalt chromium alloys, or other suitable metallic materials. In most instances, the rods **150, 152** will be located on the same side of the subject, i.e., they will be located on the same side of a plane defined by the spinous processes of the vertebrae of the subject. As such; the dimensions of the connector **100** should remain relatively small; according to the invention, the distance between the centers of the recesses **111, 129** is between about 8,9mm (0.35 inch) and about 30,5mm (1.2 inches). This distance can reduced somewhat by the nesting of the mating projections **114, 132** within complimentary recesses in the body portions **104, 122.** The depth of the recesses **111, 129** (from the front of the recess to its rear) is preferably between about 7,6mm (0,3inch) and 25.4mm (1 inch).

Those skilled in this art will recognize that alternative embodiments of the connector that differ from that illustrated herein may also be suitable. For example, the serrations **115, 133** may be replaced with roughened surfaces, knurls, or other nesting topography that prevents relative rotative movement. The pairs of set screws **112** may be replaced with a single set screw, bolts or other retaining members. The bolt **138** may be replaced with a screw, a bolt/nut combination, or another fastener.

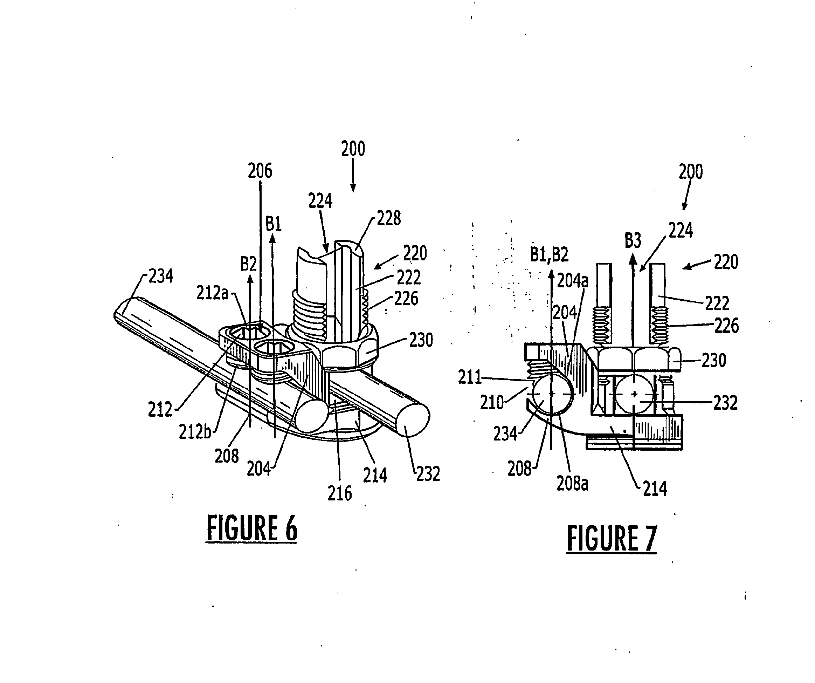

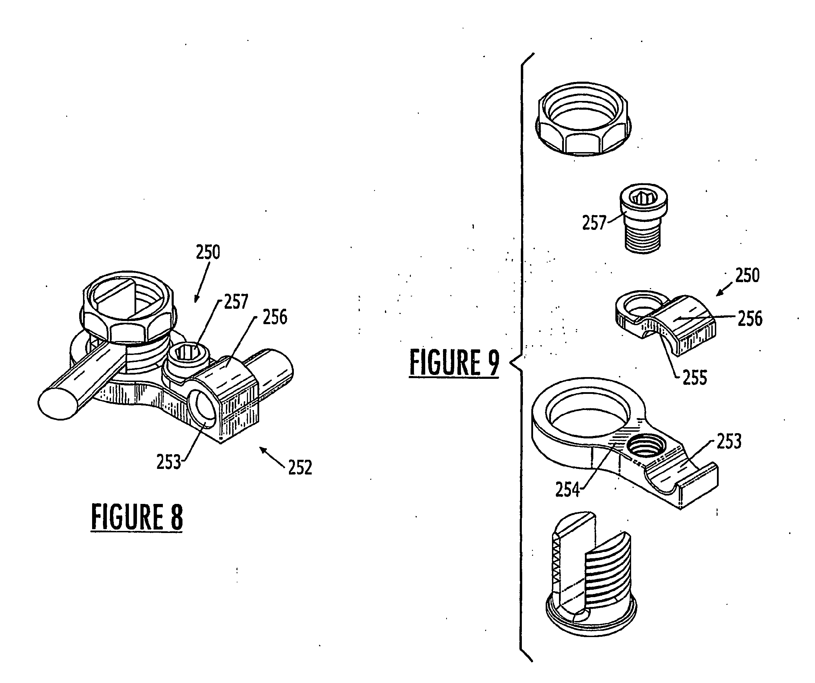

Turning now to **Figures 6** and **7**, an example of a connector which is not port of the present invention, designated broadly at **200,** is illustrated therein. The connector **200** includes a body portion **204,** a finger **208,** and a mating projection **214.** The body portion **204** is generally wedge-shaped and includes a pair of apertures **206,** each of which has a respective longitudinal axis **B1, B2.** The finger **208** extends from one end of the body portion **204.** The free end of the finger **208** and the lower edge of the body portion **204** form an opening **210** that leads to a recess **211** defined by an arcuate lower surface **208a** of the finger **208** and a substantially flat upper surface **204a** of the body portion **204.** Two set screws **212** extend through the apertures **206;** the heads **212a** of the set screws extend above the body portion **204** when in a loosened condition, and the shanks **212b** of the set screws **212** extend into the recess **211.**

The mating projection **214** extends away from a lower region of the body portion **204** in a direction generally opposite of that of the finger **208.** The mating projection **214** includes a threaded aperture **216** having a longitudinal axis **B3** that is generally parallel with the axes **B1, B2.**

An extension shaft **220** includes a shank **222** having a slot **224** and external threads **226.** The extension shaft **220** is configured such that the threaded end of the shank **220** is threaded into the aperture **216** of the connector **200.** The illustrated extension shaft **220** is of the "break-off" variety, which includes tabs **228** that can be snapped off after insertion of a nut **230** to save space within the subject.

As illustrated in **Figures 6** and **7,** the connector **200** and the extension shaft **220** can be used to interconnect two rods **232, 234.** After the rods **232, 234** are mounted in the subject, the extension shaft **220** is threaded into the aperture **216** (within which it is freely rotatable) until the slot **224** takes an orientation that enables the rod **234** to reside therein. The rod **232** is inserted into the recess **211** through the side opening **210** and secured therein in the manner described above for the connector **100.** The nut **230** is then threaded onto the external threads **226** of the extension shaft **220** and tightened to secure the rod **234** within the slot **224** of the shank **222.** Thus, the connector **200** can interconnect two rods to provide additional stability to the spine and can be adjusted within the coronal plane. As with the connector **100,** other sequences of steps for insertion of the connector **200** and rods **232, 234** may also be suitable.

Like the connector **100,** the connector **200** can be relatively simple for the surgeon to insert and secure, as the set screws **212** and the nut **230** all face away from the spine and can be tightened conveniently by the surgeon. It also is notable that the connector **200** enables the centers of the rods **232, 234** to be located at essentially the same depth in the subject, which, as described above, may be desirable.

The connector **200** is preferably formed as a unitary component, preferably of titanium, titanium alloys (like Ti-6Al-7Nb), nickel titanium alloys, cobalt chromium alloys, or other suitable metallic materials. The dimensions are typically such that the rod centers are separated by between about 8,9 mm (0.35 inch) and about 30,5mm (1.2 inches) and the depth of the recesses is between about 7,6mm (0.3inch) and 25.4mm (1 inch).

Alternative embodiments of the connector **200** include those employing screws or bolts instead of the set screws and those having jam nuts or set screws in place of the nut **230.** Of course, in these alternative embodiments, the extension shaft may have interior threads or other projections/recesses to mate with the fastener of choice. In addition, an example of a connector which is also not part of the present invention illustrated in **Figures 8** and **9** and designated broadly at **250** includes a two piece "claw" **252** formed by a recess **253** in the body portion **254** and a recess **255** in a cover portion **256.** The claw **252** is held together by a set screw **257.** The remaining structure of the connector **250** matches that of the connector **200,** and the discussion above directed to the connector **200** and its alternative embodiments is equally applicable to the connector **250.**

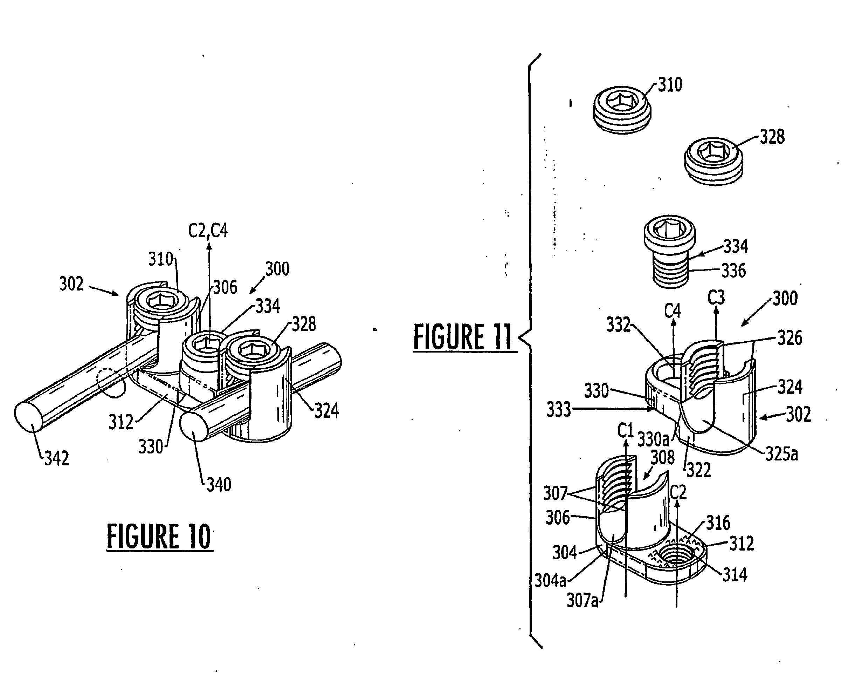

Another connector embodiment, designated broadly at **300,** is illustrated in **Figures 10** and **11.** The connector **300** includes first and second mating members **302, 320** and a bolt **334.** These components are further described below.

The first mating member **302** includes a body portion **304** to which is attached a pocket **306.** The pocket **306** is defined by side walls **307** and a floor **307a,** and has an opening **308** opposite the floor **307a.** An axis **C1** extends from the floor **307a** through the opening **308.** A set screw **310** fits within the opening **308** to close the pocket **306.** A mating projection **312** extends from a lower region **304a** of the body portion **304** in a direction away from the pocket **306.** The mating projection **312** has a threaded aperture **314** with a longitudinal axis **C2.** The mating projection **312** also has knurls **316** on its upper surface.

The second mating member **320** includes a body portion **322** and a pocket **324** formed by side walls **325** and a floor **325a.** An opening **326** is defined by the side walls **325** opposite the floor **325a;** an axis **C3** extends from the floor **325a** through the opening **326.** A set screw **328** is positioned in the opening **326** to close the pocket **324.** A mating projection **330** originates at an upper region **330a** of the body portion **322** and extends away from the pocket **324.** The mating projection **330** has an elongate aperture **332** that overlies the aperture **314;** the aperture **332** has a longitudinal axis **C4** that is generally parallel with the longitudinal axis **C2.** The mating projection **330** also has knurls **333** on its lower surface that, when the mating projection **330** overlies the mating projection **312,** mate with the knurls **316** of the mating projection **312.**

The bolt **334** is inserted through the aperture **332** and is threadedly received in the aperture **314.** The bolt **334** is sized such that its shank **336** is smaller in diameter than the width of the aperture **332.** In a loosened condition, the first and second mating members **302, 320** are free to rotate about the axes **C1, C2** and to translate laterally relative to one another (i.e. such that the distance between the pockets 306, **324** can be increased or reduced). Typically, the distance between the centers of the pockets **306, 324** will be range between about 8,9mm (0.35 inches) and about 30,5mm (1.2 inches).

In use, the connector **300** is inserted into the subject such that rods **340, 342** are secured within the pockets **306, 324.** This is accomplished by positioning the connector **300** below the rods **340, 342** (i.e., such that the pockets **306, 324** face away from the spine and toward the surgeon) without the set screws **310, 328** in place. The bolt **334** should be in a loosened condition. The connector **300** is then raised and the first and second mating members **302, 320** rotated and translated relative to each other so that the rods **340, 342** are inserted into the pockets **306, 324.** The bolt **334** is then tightened to prevent movement of the first and second mating members **302, 320** relative to each other; this movement is further prevented by the interaction of the knurls **315, 333** on the mating projections **312, 330** of the first and second mating members **302, 320.** The set screws **310, 328** are placed in the openings **308, 326** and tightened to secure the rods **340, 342** in place. Those skilled in this art will recognize that other sequences of steps for inserting the connector **300** and rods **340, 342** may also be suitable.

Those skilled in this art will recognize that alternative configurations of the connector **300** may also be suitable for use. For example, the set screws may be replaced with jam nuts or nuts threaded onto the outer surfaces of the side walls **307, 325** of the pockets **306, 324.** The bolt **334** may be replaced by a screw or other threaded fastener. The elongate aperture **332** of the mating projection **330** of the second mating member **320** may be non-elongate if relative translation of the first and second mating members **302, 320** is not required or desired. Also, alternative surface topography (such as roughened mating surfaces) may be used on the mating projections **312, 330** in place of the knurls **315, 333.**

Each of the mating members **302, 320** is typically formed as a unitary component, preferably of titanium, titanium alloys (like Ti-6Al-7Nb), nickel titanium alloys, cobalt chromium alloys, or other suitable metallic materials. In many instances, the rods **340, 342** will be located on the same side of the subject, i.e., they will be located on the same side of a plane defined by the spinous processes of the vertebrae of the subject. As such, the dimensions of the connector **300** should remain relatively small; according to the invention, the distance between the centers of the pockets **306, 324** is typically between about 8,9mm (0.35 inches) and about 30,5mm (1.2 inches).

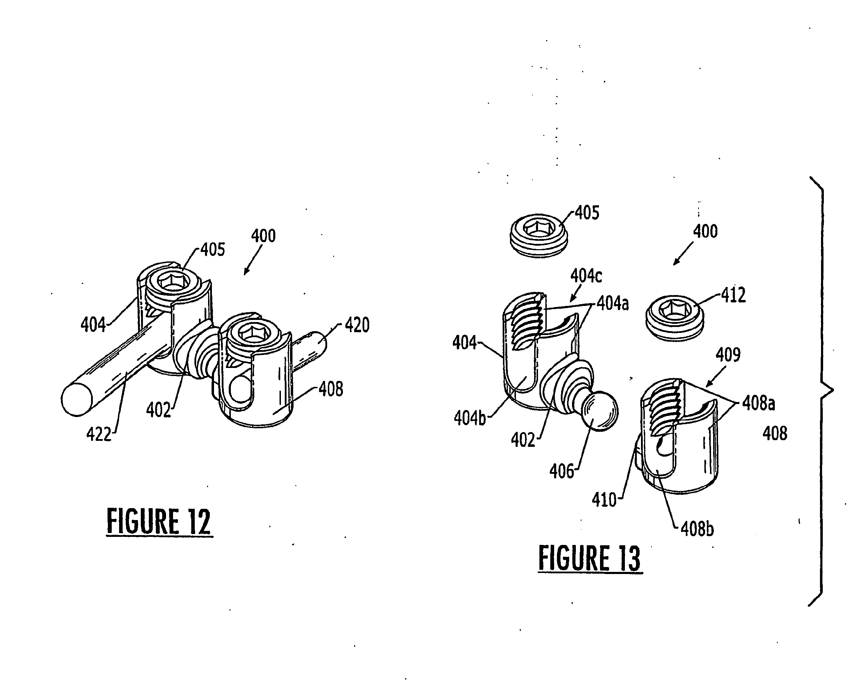

A further connector example which is not port of the present invention, designated broadly at **400**, is illustrated in **Figures 12** and **13.** The connector **400** includes a body portion **402,** a stationary pocket **404,** and a rotatable pocket **408.** The stationary pocket **404,** which is located at one end of the body portion **402,** is defined by side walls **404a** and a floor **404b;** the side walls **404a** define an opening **404c** that is opposite the floor **404b.** A set screw **405** is received within and covers the opening **404c.**

The body portion **402** includes a ball **406** at the end thereof opposite the stationary pocket **404.** The rotatable pocket **408** is rotatably attached to the ball **406** through a socket **410** that enables the pocket **408** to rotate relative to the body portion **402** about multiple axes of rotation. The rotatable pocket **408** also includes side walls **408a** and a floor **408b;** the side walls **408a** define an opening **409** opposite the floor **408b.** A set screw **412** is received within and covers the opening **409.**

In operation, the connector **400** is disposed in the subject in the same manner as the connector **300;** i.e., it is positioned such that the body portion **402** resides nearer the spine of the subject than the openings **404c, 409** of the pockets **404, 408.** With the set screws **405, 412** removed from the openings **404c, 409,** rods **420, 422** can be inserted within the pockets **404, 408,** with the rotatable pocket **408** being capable of rotating to different orientations relative to the stationary pocket **404** to adjust for the location and/or angle of the rod **422** relative to the rod **420.** Once the rods **420, 422** are inserted into their respective pockets **404, 408,** they can be secured by the insertion and tightening of the set screws **405, 412** in the openings **404c, 409;** tightening of the set screw **412** also should prevent further rotation of the rotatable pocket **408** relative to the body portion **402.** The orientation of the connector **400** can should cause the set screws **405, 412** to face the surgeon, thereby facilitating their tightening and, consequently, the securing of the connector **400** to the rods **420, 422.** As with the other connector embodiments discussed above, the sequence of steps for inserting the connector **400** and rods **420, 422** in the subject may be varied.

Those skilled in this art will recognize that alternative configurations of the connector **400** may also be suitable for use. For example, the set screws may be replaced with jam nuts or nuts threaded onto the outer surfaces of the side walls **404a, 408a** of the pockets **404, 408.** Also, the ball **406** may take a different configuration, such as one that enables the rotatable pocket to be "locked" into preferred orientations.

Typically, the body portion **402** (with the ball **406**) and the stationary pocket **404** are formed as one unitary component, while the rotatable pocket **408** is formed as a separate component. Both of these components are preferably formed of titanium, titanium alloys (like Ti-6Al-7Nb), nickel titanium alloys, cobalt chromium alloys, or other suitable metallic materials. The dimensions of the connector **400** will typically be such that the distance between the centers of the pockets **404, 408** is between about 8,9mm (0.35 inches) and about 30,5mm (1.2 inches), with the rotatable pocket **408** being able to pivot over a range of about 0 to about 270 degrees in the coronal plane, about 0 to about **360** degrees in the sagittal plane, and about 0 to about 360 degrees in the transverse plane.

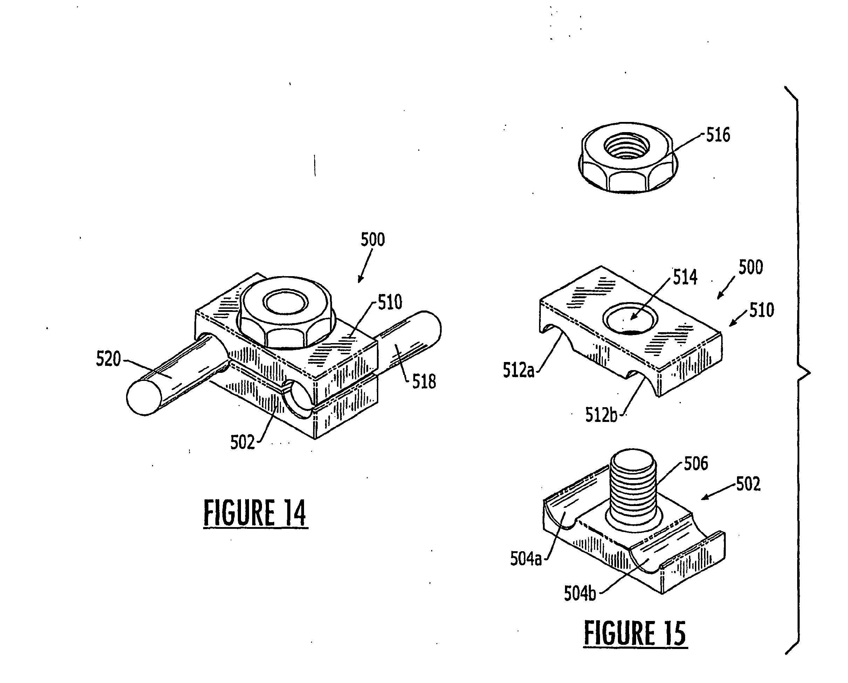

Still another connector example which is not port of the present invention, designated broadly at **500,** is illustrated in **Figures 14** and **15.** The connector **500,** which is a non-adjustable connector (i.e., with respect to rotation and lateral translation), includes a body portion **502,** a cover portion **510,** and a nut **516.** The body portion **502** has a pair of upwardly-facing recesses **504a, 504b** located on opposite sides thereof. An externally threaded post **506** rises from the center of the body portion **502.** The cover portion **510** includes a pair of downwardly-facing recesses **512a, 512b** and a centrally-located aperture **514.** The nut **516** is internally threaded and sized to receive the post **506.**

In use within a subject, the body portion **502** is positioned to underlie two rods **518, 520** attached to the spine of a patient, with the rods **518, 520** being positioned in respective recesses **504a, 504b.** The cover portion **510** is then positioned to overlie the body portion **502** so that the post **506** extends through the aperture **514** and the recesses **512a, 512b** overlie the rods **518, 520.** The nut **516** is then threaded onto the post 506 to secure the rods **518, 520** within the connector **500.**

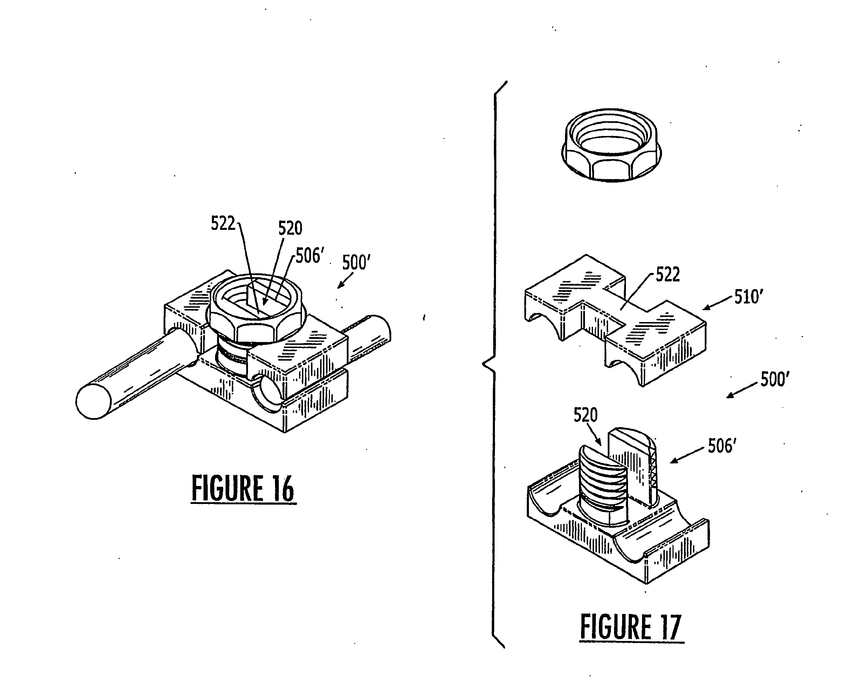

As an alternative configuration to that of the post **506** and aperture **514,** as shown in **Figures 16** and **17** in a connector **500',** the post **506'** can include a slot **520,** and the cover portion **510'** can include a bridge **522** that is received within the slot **520.**

The body portion **502** and the cover portion **510** are typically formed as unitary components and preferably of titanium, titanium alloys (like Ti-6Al-7Nb), nickel titanium alloys, cobalt chromium alloys, or other suitable metallic materials. The distance between the centers of the rods **518, 520** is preferably between about 8,9mm (0.35 inches) and about 30,5mm (1.2 inches).

Those skilled in this art will appreciate that, although the connectors of the present invention are illustrated herein for use in spinal correction, they may also be used to correct or supplement other orthopedic procedures. Also, although such procedures are typically performed on human subjects, veterinary surgeries also may benefit from the use of these connectors.

The foregoing is illustrative of the present invention and is not to be construed as limiting thereof. Although exemplary embodiments of this invention have been described, those skilled in the art will readily appreciate that many modifications are possible in the exemplary embodiments without materially departing from the novel teachings and advantages of this invention. Accordingly, all such modifications are intended to be included within the scope of this invention as defined by the appended claims.

## Claims

1. An apparatus for connecting vertebrae of a subject, comprising:
at least two rods (150,152,340,342), each of the rods defining a longitudinal axis; and
a connector (100, 300) that interconnects the rods, the connector comprising:
first (102,302) and second (120,320) mating members, each of the members including a body portion (104,122,304,322), a mating projection (114,132,312,330) and a recess (111,129,306,324) adapted to engage a respective one of the rods, the body portion including an aperture (106,124,306,324) having a longitudinal axis that is generally perpendicular to the longitudinal axes of the rods, each of the mating projections of the first and second mating members including an aperture (116,134,314,332), the mating projection of the first mating member overlying the mating projection of the second mating member such that their respective apertures are generally axially aligned;
first and second retaining members (112,130,310,328) inserted into, respectively, the body portion apertures of the first and second mating members to engage a respective rod; and
a fastener (138,334) inserted through the mating projection apertures of the first and second mating members;
wherein, when the fastener is in a tightened condition, the first and second mating members are prevented from relative rotation, and when the fastener is in a loosened condition, the first and second mating members are free to rotate about an axis of rotation that is generally parallel to the longitudinal axes of the body portion apertures of the first and second mating members; and
wherein each of the body portion recesses has a center, and wherein the recess centers are positioned between about 8,9mm (0.35 inches) and about 30,5mm (1.2 inches) from each other.

2. The apparatus defined in Claim 1, wherein contacting surfaces (115,133,316,333) on the mating projections of the first and second mating members include nesting topography that inhibits relative rotation of the first and second mating members when the fastener is in the tightened condition.

3. The apparatus defined in Claim 1, wherein the mating projection apertures are configured such that, when the fastener is in the loosened condition, the first and second mating members are free to translate relative to each other along a translation axis that is perpendicular to the axis of rotation and the longitudinal axes of the fingers of the first and second mating members.

4. The apparatus defined in Claim 1, wherein the first and second retaining members are threaded fasteners accessible from a first side of the connector, and wherein the fastener is accessible from the first side of the connector.

5. The apparatus defined in Claim 1, wherein each of the first and second mating members further comprises a finger (108,126), the fingers and the body portions of the first and second mating members together forming, respectively, the recesses of the first and second mating members.

6. The apparatus defined in Claim 1, wherein each of the body portions has an upper surface, and the mating projections of the first and second mating members are located at different first and second distances from their respective body portion upper surfaces.

7. The apparatus defined in Claim 6, wherein each of the body portion recesses has a center, and wherein the recess centers are positioned at substantially the same distance from the body portion upper surfaces.

8. The apparatus defined in Claim 1, wherein each of the body portions of the first and second mating members has a cutaway portion that is complimentary to and nested with an end of the mating projection of the other of the first and second mating members.

9. A connector (100,300) for interconnecting bone fixation rods, comprising:
first (102,302) and second (120,320) mating members, each of the members including a body portion (104,122,304,322), a mating projection (114,132,312,330) and a recess (111,129,306,324) adapted to engage a respective one of at least two bone fixation rods, the body portion including an aperture (106,124,306,324) having a longitudinal axis that is generally perpendicular to longitudinal axes of the rods, each of the mating projections of the first and second mating members including an aperture (116,134,314,332), the mating projection of the first mating member overlying the mating projection of the second mating member such that their respective apertures are generally axially aligned;
first and second retaining members (112,130,310,328) inserted into, respectively, the body portion apertures of the first and second mating members to engage a respective rod; and
a fastener (138,334) inserted through the mating projection apertures of the first and second mating members;
wherein, when the fasteners is in a tightened condition, the first and second mating members are prevented from relative rotation, and when the fastener is in a loosened condition, the first and second mating members are free to rotate about an axis of rotation that is generally parallel to the longitudinal axes of the body portion apertures of the first and second mating members; and
wherein each of the body portion recesses has a center, and wherein the recess centers are positioned between about 8,9mm (0.35 inches) and about 30,5mm (1.2 inches) from each other.

## Patentansprüche

1. Eine Vorrichtung für die Verbindung von Wirbeln eines Subjekts, die folgendes umfasst:
mindestens zwei Stäbe (150, 152, 340, 342), wobei jeder der Stäbe eine Längsachse bestimmt; und
ein Halteelement (100, 300), das die Stäbe miteinander verbindet, wobei das Halteelement folgendes umfasst:
erste (102, 302) und zweite (120, 320) Anschlusselemente, wobei jedes der Elemente einen Trägerkörperanteil (104, 122, 304, 322), einen Anschlussüberhang (114, 132, 312, 330) und eine Aussparung (111, 129, 306, 324) umfasst, dahingehend angepasst, dass sie einen entsprechenden Stab erfassen, wobei der Trägerkörperanteil eine Öffnung (106, 124, 306, 324) mit einer Längsachse enthält, die im Allgemeinen senkrecht zu den Längsachsen der Stäbe angeordnet ist, wobei jedes der Anschlussüberhänge der ersten und zweiten Anschlusselemente eine Öffnung (116, 134, 314, 332) enthält, wobei der Anschlussüberhang des ersten Anschlusselementes den Anschlussüberhang des zweiten Anschlusselementes in der Weise überlagert, dass dessen jeweilige Öffnung im Allgemeinen axial in eine Linie gebracht werden;
erste und zweite Halteelemente (112, 130, 310, 328), die jeweils zur Erfassung eines entsprechenden Stabs in die Trägerkörperanteil-Öffnungen der ersten und zweiten Anschlusselemente eingefügt sind; und
ein Befestigungselement (138, 334), das in die Anschlussüberhangsöffnungen der ersten und zweiten miteinander Anschlusselemente eingelassen ist;
wobei, wenn das Befestigungselement sich in einem angezogenen Zustand befindet, eine relative Drehung der ersten und zweiten Anschlusselemente vermieden wird, und wenn das Befestigungselement sich in einem gelockerten Zustand befindet, die ersten und zweiten Anschlusselemente sich frei um eine Drehachse drehen können, die im Allgemeinen parallel zur Längsachse der Trägerkörperanteilöffnungen der ersten und zweiten Anschlusselemente angeordnet ist; und
wobei, jede der Aussparungen der Trägerkörperanteile ein Zentrum aufweist, und wobei die Aussparungszentren zwischen in etwa 8,9 mm (0,35 Inch) und in etwa 30,5 mm (1,2 Inch) voneinander entfernt liegen.

2. Die Vorrichtung gemäß Anspruch 1, wobei Kontaktflächen (115, 133, 333) auf den Anschlussüberhängen der ersten und zweiten miteinander gepaarten Elemente eine Verschachtelungstopographie aufweisen, die eine relative Drehung der ersten und zweiten Anschlusselemente verhindert, wenn sich das Befestigungselement in einem angezogenen Zustand befindet.

3. Die Vorrichtung gemäß Anspruch 1, wobei die Anschlussüberhangöffnungen in der Weise konfiguriert sind, dass, wenn sich das Befestigungselement in einem gelockerten Zustand befindet, die ersten und zweiten Anschlusselemente frei sind, sich relativ zueinander entlang einer Verschiebungsachse zu verschieben, die senkrecht zur Drehachse und den Längsachsen der Finger der ersten und zweiten Anschlusselemente angeordnet ist.

4. Die Vorrichtung gemäß Anspruch 1, wobei die ersten und zweiten Halteelemente Befestigungselemente mit Gewinde sind, die von einer ersten Seite des Verbindungsglieds erreichbar sind, und wobei das Befestigungselement von der ersten Seite des Verbindungsglieds erreichbar ist.

5. Die Vorrichtung gemäß Anspruch 1, wobei jedes der ersten und zweiten Anschlusselemente weiterhin einen Finger (108, 126) umfasst, wobei die Finger und die Trägerkörperanteile der ersten und zweiten Anschlusselemente jeweils die Aussparungen der ersten und zweiten Anschlusselemente bilden.

6. Die Vorrichtung gemäß Anspruch 1, wobei jeder der Trägerkörperanteile eine Oberseite aufweist und die Anschlussüberhänge der ersten und zweiten Anschlusselemente in verschiedenen ersten und zweiten Abständen von ihren entsprechenden Trägerkörperanteil-Oberseiten angeordnet sind.

7. Die Vorrichtung gemäß Anspruch 6, wobei jeder der Trägerkörperanteil-Aussparungen ein Zentrum aufweist und wobei die Aussparungszentren im Wesentlichen in den gleichen Abständen von der Trägerkörperanteil-Oberseite positioniert sind.

8. Die Vorrichtung gemäß Anspruch 1, wobei jeder der Trägerkörperanteile der ersten und zweiten Anschlusselemente einen ausgeschnittenen Anteil aufweist, der komplementär zu und verschachtelt mit einem Ende des Anschlussüberhangs des anderen der ersten und zweiten Anschlusselemente ist.

9. Ein Halteelement (100, 300) zur Verbindung von Knochenfixierstäben, das folgendes umfasst:
erste (102, 302) und zweite (120, 320) Anschlusselemente, wobei jedes der Elemente einen Trägerkörperanteil (104, 122, 304, 322), einen Anschlussüberhang (114, 132, 312, 330) und eine Aussparung (111, 129, 306, 324) umfasst, dahingehend angepasst, dass sie jeweils einen der mindestens zwei Knochenfixierstäbe erfassen, wobei der Trägerkörperanteil eine Öffnung (106, 124, 306, 324) mit einer Längsachse enthält, die im Allgemeinen senkrecht zu den Längsachsen der Stäbe angeordnet ist, wobei jeder der Anschlussüberhänge der ersten und zweiten Anschlusselemente eine Öffnung (116, 134, 314, 332) enthält, wobei der Anschlussüberhang des ersten gepaarten Elementes den Anschlussüberhang des zweiten Anschlusselementes in der Weise überlagert, dass dessen jeweilige Öffnung im Allgemeinen axial in eine Linie gebracht werden;
erste und zweite Halteelemente (112, 130, 310, 328), die jeweils zur Erfassung eines entsprechenden Stabs in die Trägerkörperanteil-Öffnungen der ersten und zweiten Anschlusselemente eingefügt sind; und
ein Befestigungselement (138, 334), das in die Anschlussüberhangsöffnungen der ersten und zweiten Anschlusselemente eingelassen ist;
wobei, wenn das Befestigungselement sich in einem angezogenen Zustand befindet, eine relative Drehung der ersten und zweiten Anschlusselemente vermieden wird, und wenn das Befestigungselement sich in einem gelockerten Zustand befindet, die ersten und zweiten Anschlusselemente sich frei um eine Drehachse drehen können, die im Allgemeinen parallel zur Längsachse der Trägerkörperanteilöffnungen der ersten und zweiten Anschlusselemente angeordnet ist; und
wobei, jede der Aussparungen der Trägerkörperanteile ein Zentrum aufweist, und wobei die Aussparungszentren zwischen in etwa 8,9 mm (0,35 Zoll) und in etwa 30,5 mm (1,2 Zoll) voneinander entfernt liegen.

## Revendications

1. Dispositif pour relier les vertèbres d'un sujet, comportant :
au moins deux tiges (150, 152, 340, 342), chacune des tiges définissant un axe longitudinal ; et
un connecteur (100, 300) qui relie ensemble les tiges, ce connecteur comprenant :
des premiers organes de liaison (102, 302) et des seconds organes de liaison (120, 320), chacun des organes comprenant une portion formant corps (104, 122, 304, 322), une saillie de liaison (114, 132, 312, 330) et une cavité (111, 129, 306, 324) destinée à venir s'engager avec une des tiges respectives, la portion formant corps comprenant une ouverture (106, 124, 306, 324) ayant un axe longitudinal qui est généralement perpendiculaire aux axes longitudinaux des tiges, chacune des saillies de liaison des premiers et seconds organes de liaison comprenant une ouverture (116, 134, 314, 332), la saillie de liaison du premier organe de liaison recouvrant la saillie de liaison du second organe de liaison de telle sorte que leurs ouvertures respectives sont généralement alignées de façon axiale ;
des premiers et seconds organes de rétention (112, 130, 310, 328) engagés respectivement dans les ouvertures des portions formant corps des premiers et des seconds organes de liaison pour venir s'engager avec une tige respective ; et
un organe de fixation (138, 334) engagé au travers des ouvertures de la saillie de liaison des premiers et seconds organes de liaison;
dans lequel, lorsque l'organe de fixation est à l'état serré, les premiers et seconds organes de liaison sont empêchés de pivoter l'un par rapport à l'autre, et lorsque l'organe de fixation est à l'état desserré, les premiers et seconds organes de liaison sont libres d'effectuer une rotation autour d'un axe de rotation qui est généralement parallèle aux axes longitudinaux des ouvertures des portions formant corps des premiers et seconds organes de liaison ; et
dans lequel chacune des cavités des portions formant corps a un centre, et dans lequel les centres des cavités sont positionnés à une distance comprise entre environ 8,9 mm (0,35 pouce) et environ 30,5 (1,2 pouce) les uns des autres.

2. Dispositif défini dans la revendication 1, dans lequel les surfaces de contact (115, 133, 316, 333) sur les saillies de liaison des premiers et seconds organes de liaison incluent une topographie d'emboîtement qui empêche la rotation relative des premiers et seconds organes de liaison lorsque l'organe de fixation est à l'état serré.

3. Dispositif défini dans la revendication 1, dans lequel les ouvertures des saillies de liaison sont configurées de manière telle que, lorsque l'organe de fixation est à l'état desserré, les premiers et seconds organes de liaison sont libres d'effectuer une translation les uns par rapport aux autres le long d'un axe de translation qui est perpendiculaire à l'axe de la rotation et aux axes longitudinaux des doigts des premiers et seconds organes de liaison.

4. Dispositif défini dans la revendication 1, dans lequel les premiers et seconds organes de rétention sont des organes de fixation filetés accessibles d'un premier côté du connecteur, et dans lequel l'organe de fixation est accessible du premier côté du connecteur.

5. Dispositif défini dans la revendication 1, dans lequel chacun des premiers et seconds organes de liaison comprend de plus un doigt (108, 126), les doigts et les portions formant corps des premiers et seconds organes de liaison formant ensemble, respectivement, les cavités des premiers et seconds organes de liaison.

6. Dispositif défini dans la revendication 1, dans lequel chacune des portions formant corps a une surface supérieure, et les saillies de liaison des premiers et seconds organes de liaison sont situées à des premières et secondes distances différentes par rapport aux surfaces supérieures respectives de leurs organes de liaison.

7. Dispositif défini dans la revendication 6, dans lequel chacune des cavités de la portion formant corps a un centre, et dans lequel les centres des cavités sont positionnés substantiellement à la même distance des surfaces supérieures de la portion formant corps.

8. Dispositif défini dans la revendication 1, dans lequel chacune des portions formant corps des premiers et seconds organes de liaison a une partie coupée qui est complémentaire d'une extrémité de la saillie de liaison de l'autre parmi les premiers et seconds organes de liaison, et qui est emboîtée avec celle-ci.

9. Un connecteur (100, 300) pour relier ensemble des tiges de fixation d'os, comportant :
des premiers organes de liaison (102, 302) et des seconds organes de liaison (120, 320), chacun des organes comprenant une portion formant corps (104, 122, 304, 322), une saillie de liaison (114, 132, 312, 330) et une cavité (111, 129, 306, 324) destinée à venir s'engager avec l'une respective d'au moins deux tiges de fixation d'un os, la portion formant corps comprenant une ouverture (106, 124, 306, 324) ayant un axe longitudinal qui est généralement perpendiculaire aux axes longitudinaux des tiges, chacune des saillies de liaison des premiers et seconds organes de liaison comprenant une ouverture (116, 134, 314, 332), la saillie de liaison du premier organe de liaison recouvrant la saillie de liaison du second organe de liaison de telle sorte que leurs ouvertures respectives sont généralement alignées de façon axiale ;
des premiers et seconds organes de rétention (112, 130, 310, 328) engagés respectivement dans les ouvertures des portions formant corps du premier et du second organe de liaison pour venir s'engager avec une tige respective ; et
un organe de fixation (138, 334) engagé au travers des ouvertures des saillies de liaison des premiers et seconds organes de liaison ;
dans lequel, lorsque l'organe de fixation est à l'état serré, les premiers et seconds organes de liaison sont empêchés de pivoter l'un par rapport à l'autre, et lorsque l'organe de fixation est à l'état desserré, les premiers et seconds organes de liaison sont libres d'effectuer une rotation autour d'un axe de rotation qui est généralement parallèle aux axes longitudinaux des ouvertures des portions formant corps des premiers et seconds organes de liaison ; et
dans lequel chacune des cavités des portions formant corps a un centre, et dans lequel les centres des cavités sont positionnés à une distance comprise entre environ 8,9 mm (0,35 pouce) et environ 30,5 (1,2 pouce) les uns des autres.
